(19) Europäisches Patentamt / European Patent Office / Office européen des brevets

(11) **EP 1 638 584 B1**

(12) **FASCICULE DE BREVET EUROPEEN**

(45) Date de publication et mention
de la délivrance du brevet:
**18.08.2010 Bulletin 2010/33**

(21) Numéro de dépôt: **04742884.2**

(22) Date de dépôt: **07.06.2004**

(51) Int Cl.:
*A61K 35/20* (2006.01)    *A61L 9/013* (2006.01)
*A61P 31/14* (2006.01)    *A61P 31/20* (2006.01)
*A61P 31/22* (2006.01)    *C02F 1/68* (2006.01)
*A61K 38/17* (2006.01)

(86) Numéro de dépôt international:
**PCT/FR2004/050218**

(87) Numéro de publication internationale:
**WO 2004/110464 (23.12.2004 Gazette 2004/52)**

(54) **UTILISATION D'UNE COMPOSITION POUR INHIBER DES PARTICULES VIRALES**

VERWENDUNG EINER ZUSAMMENSETZUNG ZUR INHIBIERUNG VOM VIRUSPARTIKELN

USE OF A COMPOUND FOR INHIBITING VIRAL PARTICLES

(84) Etats contractants désignés:
**AT BE BG CH CY CZ DE DK EE ES FI FR GB GR HU IE IT LI LU MC NL PL PT RO SE SI SK TR**

(30) Priorité: **05.06.2003 FR 0306786**

(43) Date de publication de la demande:
**29.03.2006 Bulletin 2006/13**

(73) Titulaire: **INSTITUT NATIONAL DE LA RECHERCHE AGRONOMIQUE (INRA)**
**75338 Paris Cédex 07 (FR)**

(72) Inventeurs:
• **CHOBERT, Jean-Marc**
**F-44300 Nantes (FR)**
• **HAERTLE, Thomas**
**F-44240 La Chapelle sur Erdre (FR)**

(74) Mandataire: **Catherine, Alain et al**
**Cabinet Harlé & Phélip**
**7 rue de Madrid**
**75008 Paris (FR)**

(56) Documents cités:
**EP-A- 0 736 102    US-A- 5 985 275**

• SWART P J ET AL: "Charge modification of plasma and milk proteins results in antiviral active compounds." JOURNAL OF PEPTIDE SCIENCE : AN OFFICIAL PUBLICATION OF THE EUROPEAN PEPTIDE SOCIETY. DEC 1999, vol. 5, no. 12, décembre 1999 (1999-12), pages 563-576, XP0009025317 ISSN: 1075-2617 cité dans la demande
• OEVERMANN A ET AL: "The antiviral activity of naturally occurring proteins and their peptide fragments after chemical modification" ANTIVIRAL RESEARCH 01 JUN 2003 NETHERLANDS, vol. 59, no. 1, 1 juin 2003 (2003-06-01), pages 23-33, XP002269168 ISSN: 0166-3542 cité dans la demande
• SITOHY, M. ET AL: "Esterified milk proteins inhibit DNA replication in vitro" INTERNATIONAL JOURNAL OF BIOLOGICAL MACROMOLECULES , 29(4-5), 259-266 CODEN: IJBMDR; ISSN: 0141-8130, 2001, XP002269154 cité dans la demande
• FLORIS RENE ET AL: "Antibacterial and antiviral effects of milk proteins and derivatives thereof." 2003, CURRENT PHARMACEUTICAL DESIGN, VOL. 9, NR. 16, PAGE(S) 1257-1275 , XP0009025318 ISSN: 1381-6128 * p. 1264, ANTIVIRAL EFFECTS-p. 1270*
• ARNOLD DARIA ET AL: "Antiadenovirus activity of milk proteins: Lactoferrin prevents viral infection" ANTIVIRAL RESEARCH, vol. 53, no. 2, février 2002 (2002-02), pages 153-158, XP002269163 & ISSN: 0166-3542 cité dans la demande

- **SITOHY MAHMOUD ET AL: "Study of the formation of complexes between DNA and esterified dairy proteins" INTERNATIONAL DAIRY JOURNAL, vol. 11, no. 11-12, 2001, pages 873-883, XP002300102 ISSN: 0958-6946**

**Description**

La présente invention concerne le domaine général des compositions destinées à combattre les particules virales.

Ces dernières années, de nombreuses nouvelles approches ont été explorées pour lutter contre les infections virales, tel que l'utilisation d'oligonucléotides antisens, de PNAs, de dérivés de polylysine, protéines succinylées, etc ... Outre ces approches, certaines protéines cellulaires natives sont connues pour leur action inhibitrice de la réplication virale. Par exemple, il a été montré qu'une chimiokine de macrophages sous sa forme tronquée, qui est notamment produite par différents types cellulaires incluant les lymphocytes B et T, présente un large spectre inhibiteur à l'encontre de différentes souches de VIH ou SIV (Simian Immunodeficiency Virus), bien que le mécanisme d'action reste inconnu (Pal R. et al., Science 1997 ; 278 : 695 ; Struyf S. et al, J.Immunol 1998 ; 161 : 2672 ; Cota M. et al, Poli.G. PNAS 2000 ; 97 : 9162). Egalement, la protéine humaine MxA est connue pour participer à l'activité antivirale à l'encontre de plusieurs virus à ARN et ADN (Gordien E., J.Virol 2001 ; 75 : 2684).
Mais dans la grande majorité des cas, ces différentes techniques s'avèrent complexes et/ou onéreuses à mettre en oeuvre.

On a d'autre part tenté de rechercher si certains produits ou substances biologiques facilement disponibles et peu coûteuses étaient susceptibles de présenter une activité antivirale ; mais les investigations en question se sont révélées infructueuses.
En particulier, on a mis en évidence que la grande majorité des protéines connues à ce jour ne présentent pas d'activité antivirale. C'est par exemple le cas des principales protéines natives du lactosérum, tel que l'α-lactalbumine et la β-lactoglobuline, qui ne permettent pas d'inhiber l'effet cytopathique de l'*Adenovirus* (Arnold D. et al, Antiviral Res. 2002, Février ; 52 (2) : 158-8).

Pour tenter de conférer une activité anti-virale à ces protéines non actives sous forme native, des études ont été menées pour rechercher les éventuelles modifications structurelles pouvant leur apporter une telle activité.
Certaines de ces études ont montré que les protéines du lait (selon le cas les β-lactoglobuline et/ou les α-lactalbumines), modifiées par réaction avec des molécules anhydrides aromatiques conduisant à l'obtention de protéines polyanoniques, ont une activité inhibitrice sur la réplication virale ; ces résultats sont par exemple l'objet des documents US-A-5 985 275, SWART et al. (Journal of Peptides Science, Décembre 1999, volume 5, n° 12, pages 563-576), OVERMANN et al. (Antiviral Research, volume 59, n° 1, 1er Juin 2003, pages 23-33) ou FLORIS et al. (Current Pharmaceutical Design, Volume 9, n° 16, pages 1257-1275).
D'autres études ont encore mis en évidence que ces protéines du lactosérum, telles que l'α-lactalbumine et la β-lactoglobuline, sous une forme estérifiée par un alcool aliphatique (conduisant à l'obtention de protéines polycationiques), permettent une inhibition de la réplication de l'ADN par la technique PCR (M. Sitohy et al., International Journal of Biological Macromolecules ; 29(2001) 259-266); ces résultats laissaient suggérer une application potentielle de ces protéines estérifiées comme outil pour inhiber la réplication des particules virales, c'est-à-dire une utilisation pour le traitement de cellules déjà infectées (ceci est possible à condition que les protéines modifiées pénètrent les membranes cellulaires et entrent dans la cellule).

Or, la demanderesse a mis en évidence que l'utilisation préventive d'une composition comprenant au moins un dérivé d'une protéine et/ou d'un peptide dont l'un au moins de leur groupe estérifiable a été modifié par estérification, assure une inhibition significative de la capacité d'infection de particules virales contenues, ou susceptibles d'être contenues, dans un environnement particulier ; on comprend par « l'inhibition de la capacité d'infection de particules virales », l'inhibition des mécanismes permettant la pénétration desdites particules virales dans les cellules cibles en vue de leur multiplication.

L'utilisation conforme à l'invention de telles compositions ouvre de nombreuses nouvelles applications auxdites compositions, autres que celles envisagées pour les produits aptes à permettre uniquement une inhibition de la réplication des particules virales.
Ces compositions peuvent particulièrement être mises en oeuvre dans le domaine de la protection environnementale pour la lutte contre les contaminations virales, par exemple dans le traitement des eaux usées. Elles peuvent également être employées comme agents désinfectants pour le traitement des surfaces industrielles, hospitalières ou domestiques, par exemple sous forme de poudre incorporée dans des antiseptiques (savon, crème, ovule ...) ou des détergents ; les protéines estérifiées peuvent aussi être déposées à la surface des tissus de masques ou de gants de protection.
Ces applications sont particulièrement intéressantes en raison du large spectre d'activité inhibitrice de l'infection conférée par des protéines et/ou des peptides estérifiées ; par ailleurs, ces substrats protéiques estérifiés ne présentent pas de toxicité, et ils sont relativement facilement biodégradables.

Ainsi, la présente invention vise l'utilisation de compositions antivirales selon les revendications 1 ou 10, mettant en oeuvre des protéines et/ou des peptides largement disponibles et peu onéreux ; d'autre part, la technique de modification desdits peptides et/ou desdites protéines, à savoir une estérification, est largement connue, très simple à mettre en oeuvre et d'un faible coût de revient. Autre avantage, les agents antiviraux ou compositions antivirales employées présentent une innocuité environnementale et une toxicité quasi nulle. En outre, certaines compositions offrent une solution intéressante pour répondre aux problèmes d'émergence de résistances virales pouvant être rencontrés avec certains agents actifs.

La composition selon l'invention est destinée à inactiver plus particulièrement les virus à ADN ou ARN, les bactériophages ou les virinos.

De préférence, on peut encore utiliser les protéines d'origine laitière, telles que les protéines du lactosérum, notamment l'α-lactalbumine ou la β-lactoglobuline.

On notera que l'expression « peptide » utilisée ci-dessus peut définir des populations peptidiques très variées aptes à contenir diverses entités telles que des peptides, fractions peptidiques, et leurs mélanges.

Egalement, l'utilisation dans la composition, d'agents antiviraux sous forme peptidique assure une stabilité efficace de la solution, étant donné que d'une manière générale les protéines ont une plus grande tendance à précipiter.

De manière générale, on choisira de préférence des protéines et/ou des peptides dont au moins 45 % (et de préférence encore au moins 50 %) de leurs résidus acides aminés ionisables sont des résidus d'acide aspartique et/ou d'acide glutamique.

La réaction d'estérification mise en oeuvre sur le substrat protéique et/ou peptidique est conduite selon les procédures classiques, parfaitement connues de l'homme du métier (voir par exemple le procédé d'estérification en milieu acide décrit par Fraenkel-Conrat H. et Olcott H.S. 1945, J. Biol. Chem. 161, 259-269). Elle est de préférence réalisée sur au moins un groupe carboxylique du substrat de base ; elle est avantageusement conduite en milieu acide au moyen d'un alcool (ou d'un dérivé activé de cet alcool obtenu selon les méthodes classiques bien connues de l'homme du métier).

On propose l'utilisation d'un alcool aliphatique tel que le méthanol, ou l'éthanol

Dans le cadre d'une estérification par un alcool aliphatique, on obtient un substrat protéique polycationique, estérifié au niveau de son groupe carboxylique C-terminal et au niveau des groupes carboxyliques latéraux de certains des résidus d'acide aspartique et/ou d'acide glutamique. Les conditions de mise en oeuvre de la réaction d'estérification (température, durée, pression, concentration en acide ...) sont fonction du taux d'estérification désiré et, plus généralement, des produits finaux que l'on désire obtenir.

Le taux d'estérification correspondant est de préférence inférieur à 100%, avantageusement compris entre 50 et 90 %, ce qui permet une modification de type aléatoire des différents groupes estérifiables des protéines et/ou peptides concernés ; cette particularité permet l'obtention de mélanges de dérivés estérifiés de protéines et/ou de peptides, ce qui limite le risque d'apparition de souches virales résistantes, après mise en oeuvre des compositions antivirales correspondantes.

Les peptides dont l'un au moins de leurs groupes estérifiables ont été modifiés par estérification, peuvent être obtenus par tout procédé parfaitement connu à ce jour de l'homme du métier.

De préférence, ces peptides sont obtenus par la mise en oeuvre du procédé décrit dans le document EP-0 736 102. En l'espèce, le procédé correspondant consiste à réaliser une réaction d'estérification d'au moins un groupe estérifiable de l'une au moins des protéines ou peptides contenus dans le substrat d'origine animale ou végétale, dans le but de modifier l'action ultérieure d'une protéase ou peptidase.

Bien entendu, la composition comportera une concentration appropriée en protéines et/ou peptides modifiés par estérification, et cela notamment pour obtenir une activité antivirale adaptée à l'application envisagée.

Cette concentration est de préférence comprise entre 1 et 50 μg/mL.

La composition correspondante comportera tout excipient approprié, notamment pour obtenir une composition de présentation souhaitée, par exemple sous forme de liquide, solide ... ; elle comprendra encore tout principe actif, ou additif complémentaire, pour une activité optimale des dérivés protéiques et/ou peptidiques obtenus par estérification.

La présente invention a également pour objet l'utilisation préventive de certaines compositions spécifiques présentant une activité antivirale particulièrement intéressante à l'encontre de certains virus.

Ainsi, pour l'inactivation de particules virales de type *Herpes simplex,* sensible à l'acyclovir (ACV-sensible), on utilise de préférence une composition comprenant au moins un dérivé d'α-lactalbumine et/ou son hydrolysat pepsique, dont les groupes estérifiables sont méthylés entre 50 et 70 %.

D'autre part, pour l'inactivation de particules virales de type *Herpes simplex,* résistant à l'acyclovir (ACV-résistant), on emploie avantageusement une composition comprenant au moins un dérivé de β-lactoglobuline, dont les groupes estérifiables sont méthylés à au moins 90 %.

De plus, pour l'inactivation de particules virales de type *Adenovirus,* on emploie avantageusement une composition comprenant au moins un dérivé de β-lactoglobuline et/ou son hydrolysat pepsique, dont les groupes estérifiables sont méthylés à au moins 90%.

Pour l'inactivation de particules virales de type *Poliovirus,* et autres virus nus (virus d'hépatite A, *Calicivirus* ...) et enveloppés, on met en oeuvre de préférence une composition comprenant au moins un dérivé d'α-lactalbumine et/ou son hydrolysat pepsique, et/ou un dérivé de β-lactoglobuline et/ou son hydrolysat pepsique, dont les groupes estérifiables sont méthylés à au moins 60 %.

En outre, une composition utilisée pour l'inactivation de particules virales de type *Cytomegalovirus* comprend au moins un hydrolysat pepsique d'un dérivé d'α-lactalbumine, et/ou un dérivé de β-lactoglobuline, dont les groupes estérifiables sont méthylés à au moins 60 %.

Exemples

Les premières étapes des différents protocoles d'étude d'activités antivirales, c'est-à-dire les étapes visant l'obtention des protéines estérifiées et leurs hydrolysats pepsiques, sont identiques, et décrits en détail ci-dessous.

Protéines natives

La β-lactoglobuline (Protarmor 907 NK) est préparée selon la méthode de Mailliart et Ribadeau Dumas (1988, J.Food Sci. 53, 743-745).
L'α-lactalbumine (Armor Protéines) est purifiée par chromatographie d'échange d'ions sur une colonne DEAE-Sepharose Fast Flow (50 x 300 mm). L'élution est ensuite obtenue avec une solution Tris 50 mM, CaCl$_2$ 10 mM à pH 4, avec un gradient de 10 à 40 mM de NaCl.

Estérification

Le procédé d'estérification de ces α-lactalbumine et β-lactoglobuline est décrit plus en détails dans le document EP-0 736 102.
La β-lactoglobuline (5 %, rapport poids/volume) est mise en suspension dans un alcool concentré (supérieur à 99,5 %). Une quantité d'acide chlorhydrique équivalente à un rapport molaire d'acidité de 60 (mole d'acide/mole de groupe carboxylique), est ajoutée goutte-à-goutte en début de réaction. Cette préparation est ensuite placée sous agitation à 4°C pendant 8 heures. A la fin de la réaction, les échantillons sont centrifugés à 10 000 g pendant 10 minutes. Le surnageant obtenu est éliminé, et le culot est élué par agitation dans un volume d'alcool (éthanol à 99,7 %) équivalente au volume de surnageant éliminé, avant une nouvelle centrifugation sous des conditions identiques. L'étape de lavage est répétée trois fois. Le précipité final est dissous dans un volume approprié d'eau distillée, ajusté à un pH de 7,6 ; puis cette solution est dialysée contre de l'eau distillée pendant trois jours. Le pH est ensuite réajusté à 7,6 avant séchage à froid. Le lyophilisat obtenu est conservé à - 20°C en vue d'analyse. La même technique est utilisée pour l'estérification de l'α-lactalbumine, à l'exception du fait que le précipité obtenu est directement dissous dans l'eau, ajusté à un pH de 7,6, puis dialysé pendant trois jours sans lavage à l'alcool. Les étapes suivantes sont similaires à celles décrites ci-dessus pour la β-lactoglobuline.

Analyse du pourcentage d'estérification

Une réaction colorimétrique, utilisant l'hydrochlorure d'hydroxylamine, et développée par Halpin, M.I., Richardson, T. (1985, J. Dairy Sci. 68, 3189-3198), modifiée selon Bertrand-Harb, C., Chobert, J.M. Dufour, E. Haertlé, T. (1991, Sci. Aliments 11, 641-652), est mise en oeuvre pour déterminer le degré d'estérification des protéines.

Hydrolyse pepsique de protéines estérifiées

Des quantités connues de protéines natives et modifiées par estérification sont dispersées dans un tampon d'acide citrique 20 mM, pH 2,6. Des fractions aliquotées de solution de pepsine (concentration initiale : 2 mg/mL d'eau) sont ajoutées de sorte à obtenir un rapport enzyme/substrat protéique (E/S) de 2 %. Le mélange est incubé à 37°C pendant 10 heures. Un volume approprié d'une solution de phosphate disodique est ajouté pour stopper la protéolyse et pour ajuster le pH à 7,6.
Le degré de protéolyse est déterminé par quantification de l'augmentation de groupes aminés libres dans l'hydrolysat, et cela selon une réaction à l'orthophtalaldéhyde (OPA) par la mesure de l'absorption à 340 nm (Frister et al. 1988, Anal. Chem. 330 : 631-633).
Le degré d'hydrolyse (% DH) est obtenu en divisant la quantité de groupes aminés libérés par la quantité totale de groupes α-aminés liés dans le substrat de départ, puis par multiplication par 100.

Exemple 1 : Activité inhibitrice de l'infection d'une α-lactalbumine estérifiée et d'une β-lactoglobuline estérifiée à l'encontre du *Cytomegalovirus*

1.1 Mesure de l'activité anti-*Cytomegalovirus*

Une souche de référence AD 169 de *Cytomegalovirus* est multipliée au moyen de fibroblastes MRC-5 (Bio Mérieux Lyon, France). Les cellules de fibroblaste confluentes (MRC-5) sont conservées dans un milieu minimum Eagle (MEM, Eurobio) contenant 2 % de sérum foetal de veau (FCS, Eurobio).

Le milieu de culture (MEM, 2 % FCS) est ensuite retiré de l'ensemble des puits, et remplacé avec 1 mL de milieu de culture neuf dans les puits servant de contrôle cellulaire.

Dans le cas des puits servant de contrôle virus, 0,1 mL d'une suspension de virus dont le titre est connu est ajouté à 0,9 mL de milieu de culture.

Pour les puits destinés à l'évaluation de l'activité antivirale, 0,1 mL de virus et 0,1 mL d'une solution de substance testée sont ajoutés ; le volume est complété à 1 mL avec le milieu de culture.

Tous les contrôles sont répliqués quatre fois (4 puits).

Les plaques sont incubées à 37°C en présence de 5 % de $CO_2$ pendant 48 heures. Après incubation, le milieu de culture est retiré et les cellules sont fixées par addition d'acétone (à - 20°C) pendant 10 minutes. Après séchage, les cellules sont incubées avec des anticorps monoclonaux E13 à 37°C pendant 30 minutes. Après rinçage au PBS, les IgG antiglobuline, couplées avec un agent isothiocyanate fluorescent (Bio Atlantique) sont ajoutés et les cellules sont de nouveau incubées pendant 30 minutes. Pour finir, les cellules sont lavées au PBS, séchées et couvertes avec du glycérol 90 %.

Les plaques sont lues au microscope inversé (Leitz) équipé d'une lampe UV. Le nombre de noyaux fluorescents est utilisé comme index du niveau de cellules infectées par les *Cytomegalovirus.*

Le taux d'infection est calculé selon la formule suivante :

Nombre de cellules infectées (Cv) - le nombre de cellules infectées et traitées (T)/nombre de cellules infectées (Cv) x 100.

1.2 Résultats - Inhibition de la capacité d'infection à l'encontre du *Cytomegalovirus*

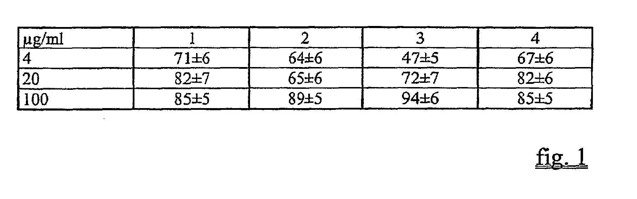

Les résultats illustrés sur la figure 1 montrent l'activité anti-infectieuse des protéines du lait estérifiées selon différentes concentrations en μg/mL (1 : α-lactalbumine méthylée à 68 % ; 2 : hydrolysat pepsique de l'α-lactalbumine méthylée à 68 % ; 3 : β-lactoglobuline méthylée à 100 % ; 4 : hydrolysat pepsique de la β-lactoglobuline méthylée à 100 %), sur la souche AD169 du *Cytomegalovirus* pendant l'opération d'infection des cellules de fibroblaste confluentes (MRC-5), après 2 jours d'incubation à 37°C.

L'α-lactalbumine méthylée et la β-lactoglobuline méthylée, ainsi que leur hydrolysat pepsique respectif, diminuent l'activité infectieuse des *Cytomegalovirus* sur les cellules de fibroblaste.

Lors de l'utilisation de faibles concentrations de protéines estérifiées (4 μg/mL), l'activité anti-infectieuse de l'α-lactalbumine est supérieure à celle de la β-lactoglobuline. Dans le cas de concentrations plus élevées de protéines estérifiées (20 et 100 μg/mL), l'activité anti-infectieuse est similaire pour les deux protéines estérifiées. De manière générale, l'activité anti-infectieuse des hydrolysats pepsique de ces protéines estérifiées est inférieure ou égale à celle de la protéine entière, à l'exception de l'hydrolysat pepsique de la β-lactoglobuline testé à faible concentration. Cependant, les formes hydrolysées de protéines estérifiées sont recommandées comme agent anti-infectieux par rapport aux protéines intactes, qui précipitent souvent.

Exemple 2: activité inhibitrice de l'infection d'une β-lactoglobuline estérifiée à l'encontre de bactériophage

2.1 Cellules et virus

Une culture de *Lactococcus lactis* IL 1403 et des solutions de bactériophages bIL 66 (21 kbp), bIL 67 (22 kbp) et bIL 170 (31 kbp) ont été fournis par Alexandra Gruss, Unité de Recherches Laitières et Génétique Appliquée, INRA, Jouy-en-Josas, France.

2.2 Mesure de l'activité anti-infectieuse des protéines estérifiées du lactosérum

Des fractions aliquotes (40 $\mu$L) de solution de protéines (de concentration initiale de 360 $\mu$M) sont mélangées avant l'infection avec 10 $\mu$L d'une solution stock de bactériophages ($10^4$-$10^5$ pfu/mL) et 10$\mu$L d'une culture en prolifération de bactéries *Lactococcus lactis* IL 1403 (D0$_{600}$ = 0,2-0,3) contenant du $CaCl_2$, 10 Mm. Ces mélanges sont incubés à 37°C, pendant 30 minutes, pour obtenir l'action maximale des protéines avant la dilution par l'addition de 100 $\mu$L de la même culture de bactéries citées (DO$_{600}$ = 0,3-0,4) et de 4 mL d'un milieu de MRS Broth contenant 0,7 % d'agar et du $CaCl_2$, 10 mM. Après incubation, le mélange est réparti à la surface d'un milieu Agar M17 contenant du glucose 0,5 %, coulé dans une boîte de Petri chauffé à 30°C. Après incubation toute une nuit, les boîtes de Petri sont étudiées pour identifier les zones d'inhibition virale.

Pour les contrôles positifs, les solutions de virus et de culture de bactéries *Lactococcus lactis* ne sont pas mélangées avec les protéines testées.

Le nombre de zones d'inhibition sur les boîtes de Petri en présence de la protéine estérifiée d'intérêt, en comparaison du contrôle positif, permet l'observation d'un éventuel effet anti-infectieux des protéines étudiées.

2.3 Résultats - Activité anti-infectieuse à l'encontre des bactériophages

Un premier essai a été mené pour étudier l'éventuelle activité anti-infectieuse de la β-lactoglobuline méthylée à 100 % à l'encontre du bactériophage bIL 66 ($10^4$ pfu/mL), en comparaison avec le contrôle positif, réalisé en absence de protéines ; la concentration en β-lactoglobuline méthylée dans le milieu de pré-incubation est de 240 $\mu$M.

Les résultats de cet essai montrent que cette β-lactoglobuline méthylée à 100 % diminue de 55 % l'activité infectieuse du bactériophage bIL 66. Lorsque les protéines sont ajoutées directement dans le milieu le plus dilué, son action est quasi inexistante voire inexistante. Par conséquent, la concentration de protéines dans le milieu initial de culture doit être suffisamment élevée pour obtenir l'effet inhibiteur sur l'activité infectieuse du virus.

Un second essai a été mené pour étudier l'effet sur l'activité infectieuse du bactériophage bIL 67 ($10^5$ pfu/mL) de la β-lactoglobuline méthylée à 100 %, en comparaison avec des témoins en l'absence de protéines ; la concentration de protéines dans le milieu de pré-incubation est de 240 $\mu$M.

Les résultats obtenus montrent une activité anti-infectieuse des protéines estérifiées similaires à l'encontre du bactériophage bIL 67. Cependant, l'activité de la β-lactoglobuline méthylée à 100 % est accrue, avec une inhibition de l'ordre de 97 % de l'activité infectieuse.

Au regard de l'ensemble de ces résultats, on observe que les protéines du lactosérum modifiées par estérification, en particulier les β-lactoglobulines estérifiées, ont un effet d'inhibition sur les mécanismes d'infection des bactériophages lactoccaux.

Exemple 3 - Activité antivirale d'une $\alpha$-lactalbumine estérifiée et d'une β-lactoglobuline estérifiée à l'encontre du virus *Herpes simplex* de type 1 (HSV-1).

3.1 Cellules et virus

Une ligne cellulaire Vero ATCC CCL81 (Cellules de rein de singe vert d'Afrique) est multipliée dans un milieu MEM (Eurobio) contenant 8 % de sérum foetal de veau (FCS, Eurobio) et 1 % de PCS (pénicilline 10 000 U, colimycine 25 000 U, streptomycine 10 mg).

Un lot de HSV-1, souche sauvage 17 ACV$^{\underline{s}}$ et PCS$^{\underline{s}}$ (HSV-1), est multiplié dans les cellules Vero et conservé à - 70°C en vue de son utilisation.

3.2 Mesure de l'activité anti-HSV-1 et essai de cytotoxicité

100 $\mu$L d'une gamme de concentration de protéines estérifiées sont dilués avec une solution de MEM contenant 8 % de FCS, puis ajoutés à chacun des puits d'une plaque 96 puits contenant chacun 50 $\mu$L d'une suspension de cellules Vero ($10^5$ cellules par mL).

Pour procéder au test d'activité anti-HSV-1, 50 $\mu$L d'une suspension de virus sont ajoutés aux cellules à une dose équivalant à un effet viral cytopathogène déterminé (CPE), en présence de différentes concentrations de la substance testée.

Pour tester la cytotoxicité, 50 $\mu$L de MEM contenant 8 % de FCS sont ajoutés aux puits en présence de différentes concentrations de protéines, sans virus.

Des contrôles cellules et virus sont menés en simultané.

Après 72 heures d'incubation à 37°C, en présence de 5 % de $CO_2$, l'activité antivirale est suivie par une évaluation de la viabilité cellulaire par la méthode du rouge neutre (Mac Laren et al. 1983, Antivirale Res. 3 : 223-234). La densité optique est directement proportionnelle à la quantité de cellules viables, qui est inversement proportionnelle au rapport CPE.

Le pourcentage de protection cellulaire est calculé selon la formule suivante :

$$\text{Protection cellulaire (\%)} = DOs - DOv/DOc - DOv \times 100$$

où DOs est la densité optique mesurée pour les cellules infectées par le virus et protégées par la substance testée ; la DOv est la densité optique mesurée pour des cellules infectées par le virus ; la DOc est la densité optique mesurée pour des cellules contrôles (sans infection par un virus, ni traitement par la substance testée).

La cytotoxicité de la substance testée est calculée par comparaison de la viabilité des cellules multipliées en présence de la substance testée par rapport aux cellules contrôles (absence de substance testée), selon la formule suivante :

$$\text{Cytotoxicité (\%)} = DOc - DOs/DOc \times 100$$

La même procédure est utilisée sur des cellules fixées par incubation pendant 24 heures, 50 $\mu$L de suspension cellulaire étant distribués dans chaque puits de la plaque. Le jour suivant, les virus et la substance testée sont ajoutés tel que défini précédemment. La plaque est incubée pendant trois jours avant de déterminer la viabilité cellulaire.

### 3.3 Résultats

#### 3.3.1 Activité antivirale de protéines estérifiées à l'encontre du virus HSV-1, ACV-sensible

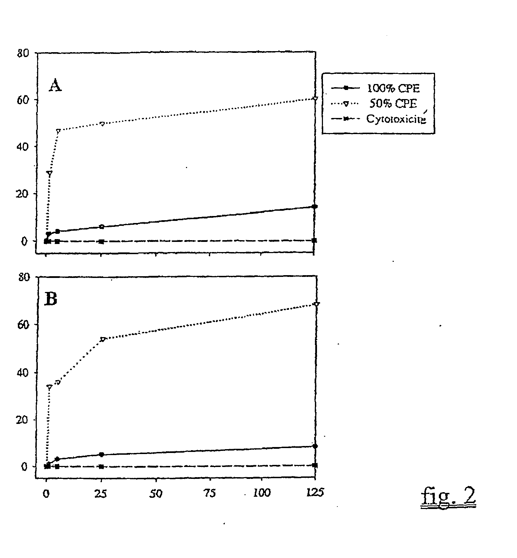

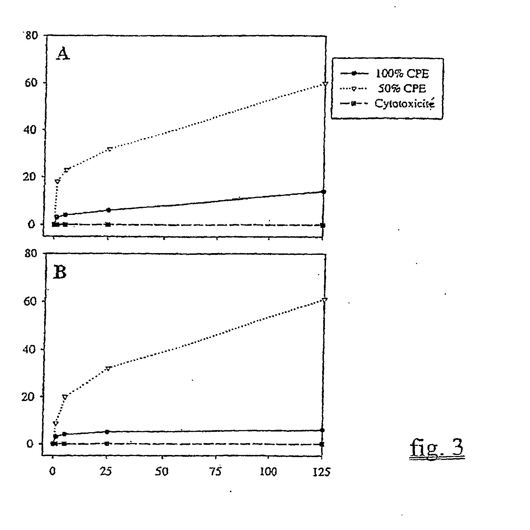

La figure 2 représente le pourcentage de protection cellulaire obtenu par l'$\alpha$-lactalbumine méthylée à 60 % (A) et son hydrolysat pepsique-10 heures (B) à l'encontre du virus HSV-1 (CPE 50 et 100 %) inoculé à des cellules Vero, après 72 heures d'incubation à 37°C, et cela en fonction de la concentration de protéines en $\mu$g/mL.
L'$\alpha$-lactalbumine méthylée à 60 %, et son hydrolysat pepsique (25 % DH ; peptides de masse moléculaire inférieure à 1 kDa), exercent une activité antivirale plus prononcée à l'encontre de faibles quantités de virus (50 % CPE). L'hydrolysat pepsique est aussi efficace que la protéine native estérifiée. La concentration moyenne de protéines permettant l'obtention d'une inhibition de 50 % ($IC_{50}$) est similaire dans le cas de la protéine estérifiée et de son hydrolysat ($20 \pm 1$ $\mu$g/mL).
Les résultats représentés sur la figure 3 montrent une activité similaire de la $\beta$-lactoglobuline méthylée à 100 % (A), et de son hydrolysat pepsique (B) (25 % DH ; peptides résultants de masse moléculaire inférieure à 1 kDa) ; l'$IC_{50}$ est de $95 \pm 1,5$ et $85 \pm 1,5$ $\mu$g/mL, respectivement.
L'activité antivirale de l'$\alpha$-lactalbumine méthylée à 60 % est supérieure à celle de la $\beta$-lactoglobuline méthylée à 100 %, en dépit de son faible degré d'estérification.
Il est à noter que ni l'$\alpha$-lactalbumine native, ni la $\beta$-lactoglobuline native, ne présentent d'activité antivirale à l'encontre du virus HSV-1 (résultats non présentés).
Dans les concentrations de protéines utilisées pour ces expérimentations (0-110 $\mu$g par mL), aucun signe de cytotoxicité n'a été observé.

#### 3.3.2 Activité antivirale de protéines estérifiées à l'encontre de souches de virus HSV-1, ACV-résistant

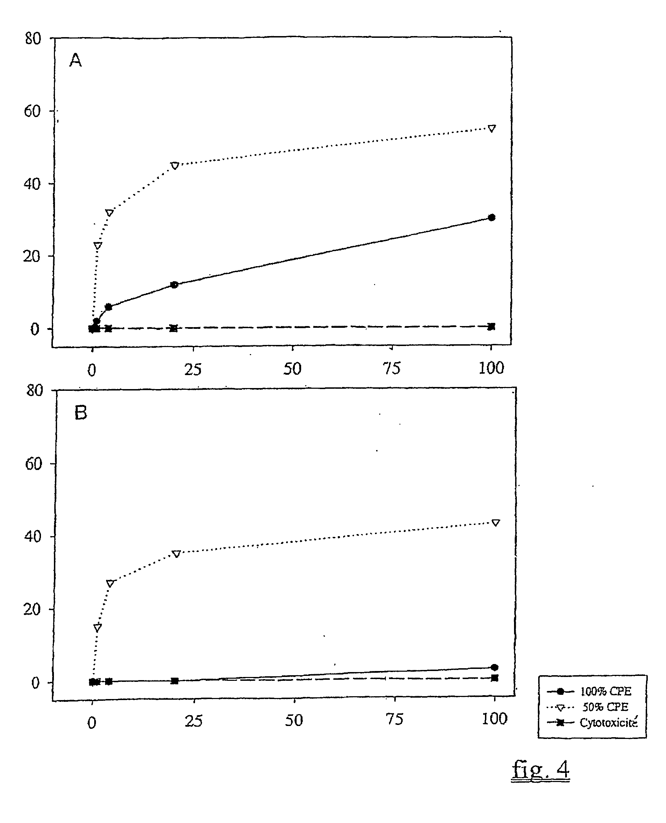

La figure 4 représente le pourcentage de protection cellulaire obtenue par l'application d'$\alpha$-lactalbumine méthylée à 60 % (A) et de son hydrolysat pepsique-10 heures (B) à l'encontre du virus HSV-1, ACV-résistant inoculé sur des cellules fixées Vero, après 72 heures d'incubation à 37°C, et cela en fonction de la concentration de protéines en $\mu$g/mL.
Les résultats correspondants montrent que l'$\alpha$-lactalbumine méthylée à 60 %, et son hydrolysat pepsique, sont tous deux actifs à l'encontre de la souche de virus HSV-1, ACV-résistante, pour une concentration inhibitrice 50 de $58 \pm 1,6$ $\mu$g/mL pour l'$\alpha$-lactalbumine méthylée à 60 %. Cette activité antivirale est légèrement inférieure à celle observée dans le cas de la souche de virus HSV-1, ACV-sensible ($IC_{50}$ : $20 \pm 1$ $\mu$g/mL). Cependant, contrairement à ce qui a été observé dans le cas de la souche HSV-1, ACV-sensible, la concentration inhibitrice 50 n'est pas mesurable avec l'hydrolysat pepsique de l'$\alpha$-lactalbumine méthylée à 60 %, dans le domaine de concentration utilisé.

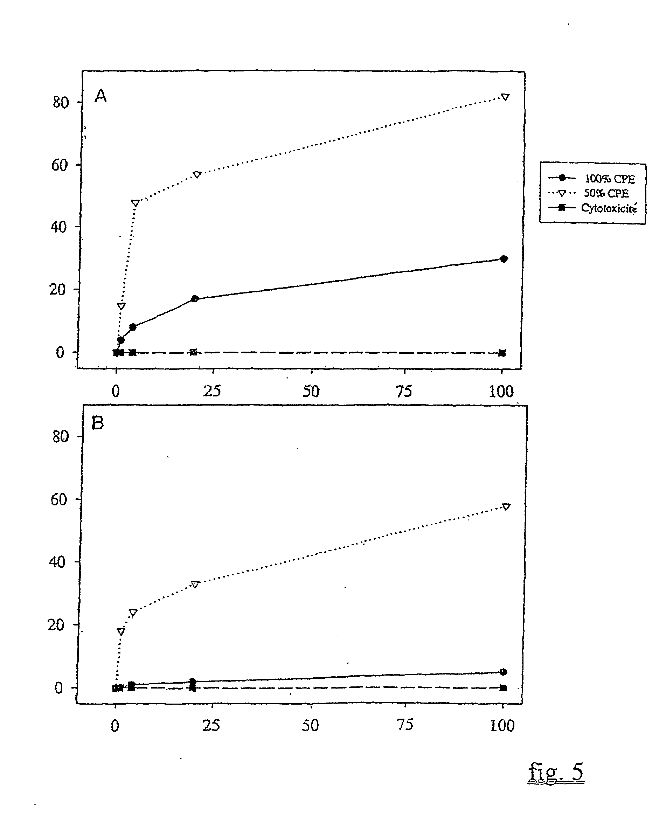

La figure 5 représente le pourcentage de protection cellulaire obtenue par la $\beta$-lactoglobuline méthylée à 100 % (A) et son hydrolysat pepsique-10 heures (B) à l'encontre du virus HSV-1, ACV-résistant, inoculés à des cellules Vero fixées, après 72 heures d'incubation à 37°C, et cela en fonction de la concentration en protéines en $\mu$g/mL.

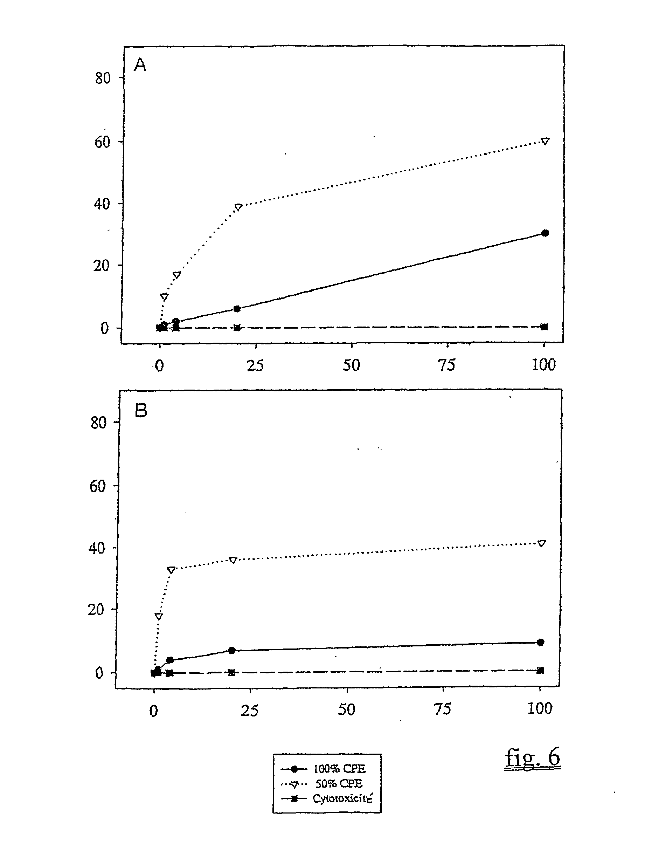

La figure 6 représente le pourcentage de protection cellulaire obtenue par l'application de β-lactoglobuline éthylée à 73 % (A) et son hydrolysat pepsique-10 heures (B) à l'encontre du virus HSV-1, ACV-résistant, inoculés sur des cellules fixées Vero, après 72 heures d'incubation à 37°C, et cela en fonction de la concentration de protéines en μg/mL.

Le même phénomène est observé pour la β-lactoglobuline méthylée et éthylée, et pour leurs hydrolysats respectifs. La concentration inhibitrice 50 est de 8 ± 1 et de 75 ± 2 μg/mL pour la β-lactoglobuline méthylée et pour son hydrolysat pepsique, respectivement, et de 62 ± 3 μg/mL pour la β-lactoglobuline éthylée.

Tel qu'observé dans le cas de l'α-lactalbumine méthylée à 60 %, la concentration inhibitrice 50 n'est pas mesurable dans le cas de l'hydrolysat pepsique de la β-lactoglobuline éthylée.

La β-lactoglobuline méthylée à 100 % présente une activité antivirale à l'encontre de la souche de virus HSV-1, ACV-résistante, supérieure à celle de l'α-lactalbumine méthylée à 60 %.

Exemple 4 : Activité antivirale d'une α-lactalbumine estérifiée et d'une β-lactoglobuline estérifiée à l'encontre du *Poliovirus*

4.1 Mesure de l'activité *anti-Poliovirus* et essai de cytotoxicité

100 μL de différentes concentrations de protéines estérifiées, ou de peptides pepsiques, dilués dans un milieu MEM, contenant 8 % de FCS, sont répartis dans des plaques 96 puits contenant 50 μL de suspension cellulaire de lignée Vero ($10^5$ cellules par ml).

Pour tester l'activité antivirale, 50 μL d'une suspension de *Poliovirus* type 1 ou *Poliovirus* type 2, à une concentration équivalente à 50 ou 100 % CPE, sont ajoutés aux cellules, en présence de différentes concentrations de protéines estérifiées ou de peptides pepsiques.

Pour tester la cytotoxicité, 50 μL de différentes concentrations de protéines estérifiées ou de peptides pepsiques, dilués dans une solution MEM contenant 8 % de FCS, sont ajoutés aux cellules en présence de virus.

Des contrôles cellules et contrôles virus sont effectués simultanément.

Après 48 heures (dans le cas de *Poliovirus* type 1) ou 96 heures (dans le cas de *Poliovirus* type 2), d'incubation à 37°C en présence de 5 % de $CO_2$, l'activité antivirale est étudiée par l'évaluation de la viabilité des cellules par la méthode au rouge neutre (Mac Laren et al. 1983), tel que décrit précédemment lors de la mesure de l'activité anti HSV-1 des protéines estérifiées, au titre 3.2.

4.2 : Résultats - Activité antivirale à l'encontre des *Poliovirus*

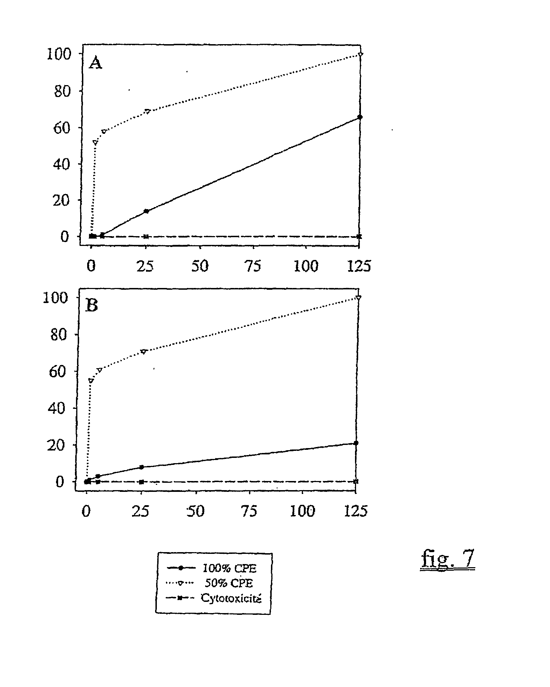

La figure 7 illustre le pourcentage de protection cellulaire de l'α-lactalbumine méthylée à 68 % (A), et de son hydrolysat pepsique-10 heures (B), à l'encontre du *Poliovirus* type 1, de titre 50 et 100 % CPE, inoculé à des cellules Vero, après 48 heures d'incubation à 37°C, et cela en fonction de la concentration de protéines en μg/mL.

L'α-lactalbumine méthylée à 68 %, et son hydrolysat pepsique (25 % DH, peptides obtenus de masse moléculaire inférieure à 1 kDa), montrent une protection cellulaire considérable et similaire, à l'encontre de la plus faible concentration en virus (50 % CPE). La concentration moyenne en protéines permettant l'obtention d'une inhibition à 50 % ($IC_{50}$) est de l'ordre de 0,8 ± 0,1 μg/mL dans les deux cas. L'activité antivirale est généralement inférieure à l'encontre d'une concentration supérieure en virus (100 % CPE), où l'α-lactalbumine entière méthylée à 68 % est plus efficace que sa forme hydrolysée.

Aucun signe de cytotoxicité n'a été observé dans le domaine de concentration testé (1 à 125 μg par mL).

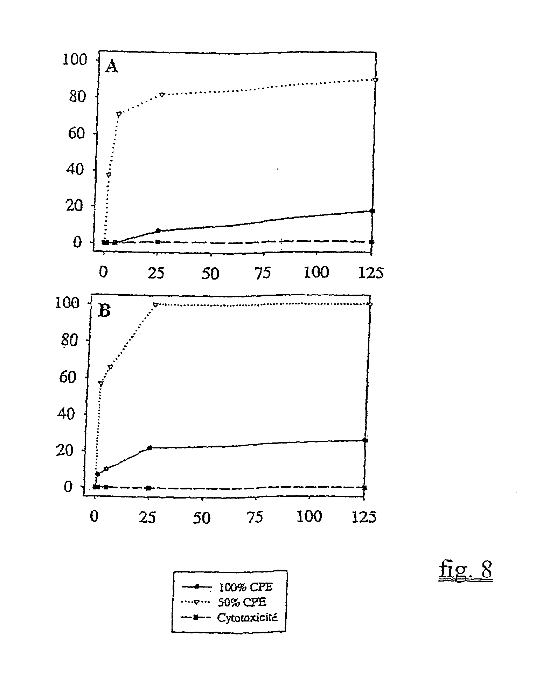

La figure 8 montre le pourcentage de protection cellulaire obtenue par l'application de β-lactoglobuline méthylée à 100 % (A) et son hydrolysat pepsique-10 heures (B) à l'encontre de *Poliovirus* type 1 inoculé sur des cellules Vero, après 48 heures d'incubation à 37°C, et cela en fonction de la concentration de protéines d'intérêt en μg/mL.

Les informations correspondantes montrent des résultats similaires dans le cas de la β-lactoglobuline méthylée à 100 % (A), et de son hydrolysat pepsique-10 heures (B) (25 % DH, peptide résultant de masse moléculaire inférieure à 1 kDa), avec une $IC_{50}$ de l'ordre de 0,8 ± 0,1 μg/mL pour le titre inférieur en virus (50 % CPE).

Bien que l'action protectrice pour un titre supérieur en virus (100 % CPE) soit relativement inférieure à celle présentée ci-dessus, il n'y a pas de différence significative entre l'action de la β-lactoglobuline méthylée sous sa forme entière et sous sa forme hydrolysée.

L'α-lactalbumine et la β-lactoglobuline, sous leur forme native non modifiée, n'ont pas d'activité antivirale à l'encontre des *Poliovirus* type 1 (résultats non présentés).

Une activité antivirale similaire est observée pour les deux protéines estérifiées, et leurs hydrolysats pepsiques respectifs, à l'encontre de *Poliovirus* type 2 infectant des cellules Vero incubées pendant 4 jours à 37°C. Cependant, l'activité antivirale est légèrement inférieure à celle observée dans le cas de *Poliovirus* type 1 (résultats non présentés). La valeur de l'$IC_{50}$ mesurée pour un titre de virus de 50 % CPE est de 8 ± 0,5 μg/mL pour l'α-lactalbumine méthylée à 68 %, et de 6 ± 0,4 μg/mL pour son hydrolysat pepsique-10 heures. D'autre part, la valeur de $IC_{50}$ (50 % CPE) est

de 6 $\pm$ 0,4 $\mu$g/mL pour la β-lactoglobuline méthylée à 100 %, et de 3 $\pm$ 0,2 $\mu$g/mL pour son hydrolysat pepsique-10 heures.
La β-lactoglobuline éthylée à 70 % présente aussi une activité antivirale à l'encontre de *Poliovirus* type 2 avec une $IC_{50}$ de 10 $\pm$ 0,6 $\mu$g/mL pour un titre de virus de 50 % CPE (résultats non représentés).

Exemple 5 : Activité antivirale d'une α-lactalbumine estérifiée et d'une β-lactoglobuline estérifiée à l'encontre de l'*Adenovirus*

5.1 Mesure de l'activité *anti-Adenovirus* et essai de cytotoxicité

La méthode suivie pour l'étude de l'activité antivirale des protéines estérifiées à l'encontre d'*Adenovirus* est similaire à celle décrite précédemment en relation avec l'exemple 4, au point 4.1 ; ces tests sont ici mis en oeuvre sur une ligne cellulaire Heps incubée à 37°C pendant 48 à 72 heures.

5. 2 : Résultat : Activité antivirale à l'encontre de l'*Adenovirus*

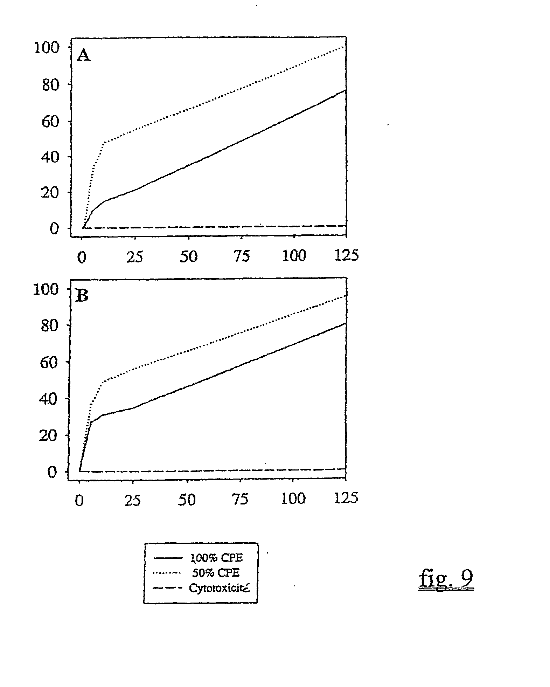

La figure 9 représente le pourcentage de protection cellulaire obtenue par l'α-lactalbumine méthylée à 68 % (A) et son hydrolysat pepsique-10 heures (B) à l'encontre d'*Adenovirus* inoculé sur des cellules Heps, après 48 heures d'incubation à 37°C, et cela en fonction de la concentration de protéines en $\mu$g/mL.

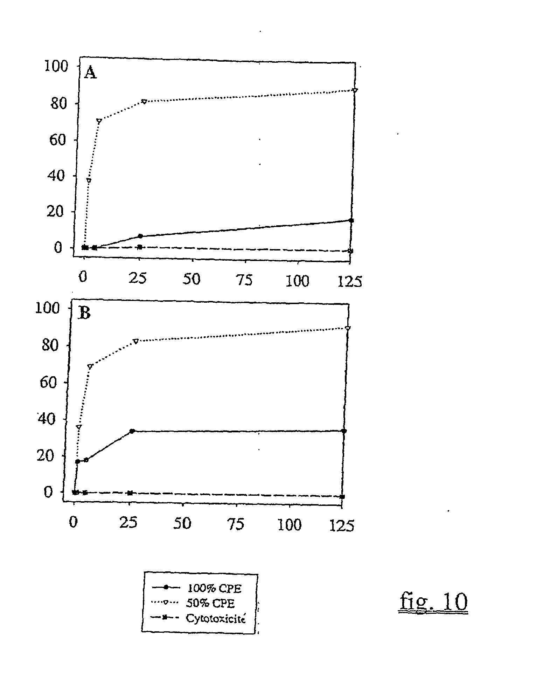

La figure 10 illustre le pourcentage de protection cellulaire obtenue par l'application de β-lactoglobuline méthylée à 100 % (A) et son hydrolysat pepsique-10 heures (B) à l'encontre d'*Adenovirus* inoculé sur des cellules Heps, après 48 heures d'incubation à 37°C, et cela en fonction de la concentration de protéines appliquées en $\mu$g/ml.
Les résultats correspondants montrent que l'α-lactalbumine méthylée à 68 % et la β-lactoglobuline méthylée à 100 %, ainsi que leur hydrolysat pepsique-10 heures, sont actifs à l'encontre d'*Adenovirus* infectant des cellules fixées Heps, après 48 heures d'incubation à 37°C. Ces activités sont directement en relation avec la concentration en protéines et peptides testés, et cela de manière encore plus évidente à faible concentration de virus (50 % CPE). Il n'y a pas différence significative, en terme d'activité antivirale, entre les protéines entières modifiées et leurs formes hydrolysées. L'α-lactalbumine méthylée à 68 %, et son hydrolysat pepsique, inhibent l'activité de l'*Adenovirus,* avec une $IC_{50}$ de 11 $\pm$ 0,6 $\mu$g/mL (50 % CPE). La $IC_{50}$ pour la β-lactoglobuline méthylée à 100 %, et son hydrolysat pepsique, est de l'ordre de 3,5 $\pm$ 0,2 $\mu$g/mL.

**Revendications**

1. Utilisation préventive non thérapeutique, dans un environnement contenant ou susceptible de contenir des particules virales, à l'exclusion des environnements in-vivo, d'une composition pour inhiber au moins partiellement les mécanismes d'infection desdites particules virales, laquelle composition comprend au moins une protéine choisie parmi l'α-lactalbumine et/ou la β-lactoglobuline, et/ou un peptide provenant d'une telle protéine, dont l'un au moins de leurs groupes estérifiables a été modifié par estérification en présence de méthanol ou d'éthanol.

2. Utilisation conforme à la revendication 1, dans laquelle la composition comprend au moins un dérivé d'α-lactalbumine et/ou son hydrolysat pepsique, dont les groupes estérifiables sont méthylés entre 50 et 70 %, pour inhiber les capacités d'infection de particules virales de type *Herpes Simplex* acyclovir-sensible.

3. Utilisation conforme à la revendication 1, dans laquelle la composition comprend au moins un dérivé de β-lactoglobuline, dont les groupes estérifiables sont méthylés à au moins 90 %, pour l'inhibition des capacités d'infection de particules virales de type *Herpes Simplex* acyclovir-Résistant.

4. Utilisation conforme à la revendication 1, dans laquelle la composition comprend au moins un dérivé de β-lactoglobuline et/ou son hydrolysat pepsique, dont les groupes estérifiables sont méthylés à au moins 90 %, pour inhiber les capacités d'infection de particules virales de type *Adenovirus.*

5. Utilisation conforme à la revendication 1, dans laquelle la composition comprend au moins un dérivé d'α-lactalbumine et/ou son hydrolysat pepsique, et/ou un dérivé de la β-lactoglobuline et/ou son hydrolysat pepsique, dont les groupes estérifiables sont méthylés à au moins 60 %, pour inhiber les capacités d'infection de particules virales de type *Poliovirus* et/ou autres virus nus et enveloppés.

6. Utilisation conforme à la revendication 1, dans laquelle la composition comprend au moins un hydrolysat pepsique d'un dérivé d'α-lactalbumine, et/ou un dérivé de β-lactoglobuline, dont les groupes estérifiables sont méthylés à au moins 60 %, pour inhiber les capacités d'infections de particules virales de type *Cytomegalovirus.*

7.  Utilisation préventive d'une composition conforme à l'une quelconque des revendications 1 à 6, comme agent désinfectant pour le traitement des surfaces, par exemple sous forme de poudre, incorporée dans des antiseptiques ou des détergents.

8.  Utilisation préventive d'une composition conforme à l'une quelconque des revendications 1 à 6, comme agent pour la lutte contre les contaminations virales dans le traitement des eaux usées.

9.  Utilisation préventive d'une composition conforme à l'une quelconque des revendications 1 à 6, comme agent antiviral déposé à la surface des tissus de masques ou de gants de protection.

10. Utilisation d'au moins une protéine choisie parmi l'α-lactalbumine et/ou la β-lactoglobuline, et/ou un peptide provenant d'une telle protéine, dont l'un au moins de leurs groupes estérifiables a été modifié par estérification en présence de méthanol ou d'éthanol, pour la fabrication d'une composition assurant une inhibition au moins partielle des mécanismes d'infection de particules virales.

11. Utilisation conforme à la revendication 10 d'au moins un dérivé d'α-lactalbumine et/ou son hydrolysat pepsique, dont les groupes estérifiables sont méthylés entre 50 et 70 %, pour la fabrication d'une composition pour inhiber les capacités d'infection de particules virales de type *Herpes Simplex* acyclovir-Sensible.

12. Utilisation conforme à la revendication 10 d'au moins un dérivé de β-lactoglobuline, dont les groupes estérifiables sont méthylés à au moins 90 %, pour la fabrication d'une composition pour l'inhibition des capacités d'infection de particules virales de type *Herpes Simplex* acyclovir-Résistant.

13. Utilisation conforme à la revendication 10 d'au moins un dérivé de β-lactoglobuline et/ou son hydrolysat pepsique, dont les groupes estérifiables sont méthylés à au moins 90 %, pour la fabrication d'une composition permettant l'inhibition des capacités d'infection de particules virales de type *Adenovirus.*

14. Utilisation conforme à la revendication 10 d'au moins un dérivé d'α-lactalbumine et/ou son hydrolysat pepsique, et/ou un dérivé de la β-lactoglobuline et/ou son hydrolysat pepsique, dont les groupes estérifiables sont méthylés à au moins 60 %, pour la fabrication d'une composition permettant l'inhibition des capacités d'infection de particules virales de type *Poliovirus* et/ou autres virus nus et enveloppés.

15. Utilisation conforme à la revendication 10 d'au moins un hydrolysat pepsique d'un dérivé d'α-lactalbumine, et/ou un dérivé de β-lactoglobuline, dont les groupes estérifiables sont méthylés à au moins 60 %, pour la fabrication d'une composition permettant l'inhibition des capacités d'infection de particules virales de type *Cytomegalovirus.*

**Claims**

1.  Non-therapeutic preventive use, in an environment containing or which may contain viral particles, excluding in vivo environments, of a composition for inhibiting at least partially the infection mechanisms of said viral particles, which composition comprises at least one protein selected from α-lactalbumin and/or β-lactoglobulin, and/or a peptide from such a protein, at least one of their esterifiable groups of which has been modified by esterification in the presence of methanol or ethanol.

2.  Use according to claim 1, wherein the composition comprises at least a α-lactalbumin derivative and/or its pepsin hydrolysate, the esterifiable groups of which are methylated between 50 and 70%, for inhibiting the infection capacities of viral particles of the acyclovir-sensible *Herpes Simplex* type.

3.  Use according to claim 1, wherein the composition comprises at least a β-lactoglobulin derivative, the esterifiable groups of which are methylated to at least 90%, for the infection capacity inhibition of viral particles of the acyclovir-resistant *Herpes Simplex* type.

4.  Use according to claim 1, wherein the composition comprises at least a β-lactoglobulin derivative and/or its pepsin hydrolysate, the esterifiable groups of which are methylated to at least 90%, for inhibiting the infection capacities of viral particles of the *Adenovirus* type.

**5.** Use according to claim 1, wherein the composition comprises at least a α-lactalbumin derivative and/or its pepsin hydrolysate, and/or a β-lactoglobulin derivative and/or its pepsin hydrolysate, the esterifiable groups of which are methylated to at least 60%, for inhibiting the infection capacities of viral particles of the *Poliovirus* type and/or other naked and enveloped viruses.

**6.** Use according to claim 1, wherein the composition comprises at least a pepsin hydrolysate of a α-lactalbumin derivative, and/or a β-lactoglobulin derivative, the esterifiable groups of which are methylated to at least 60%, for inhibiting the infection capacities of viral particles of the *Cytomegalovirus* type.

**7.** Preventive use of a composition according to any one of claims 1 to 6, as a disinfecting agent for the treatment of surfaces, for example in a powder form, incorporated into antiseptics or detergents.

**8.** Preventive use of a composition according to any one of claims 1 to 6, as an agent for controlling the viral contaminations in the treatment of the wastewater.

**9.** Preventive use of a composition according to any one of claims 1 to 6, as an antiviral agent deposited on the surface of tissue of protective masks or gloves.

**10.** Use of at least one protein selected from α-lactalbumin and/or β-lactoglobulin, and/or a peptide from such a protein, at least one of their esterifiable groups of which has been modified by esterification in the presence of methanol or ethanol, for the manufacture of a composition ensuring an at least partial inhibition of the viral particle infection mechanisms.

**11.** Use according to claim 10 of at least a α-lactalbumin derivative and/or its pepsin hydrolysate, the esterifiable groups of which are methylated between 50 and 70%, for the manufacture of a composition to inhibit the infection capacities of viral particles of the acyclovir-sensible *Herpes Simplex* type.

**12.** Use according to claim 10 of at least a β-lactoglobuline derivative, the esterifiable groups of which are methylated to at least 90%, for the manufacture of a composition for inhibiting the infection capacities of viral particles of the acyclovir-resistant *Herpes Simplex* type.

**13.** Use according to claim 10 of at least a β-lactoglobuline derivative and/or its pepsin hydrolysate, the esterifiable groups of which are methylated to at least 90%, for the manufacture of a composition allowing the infection capacity inhibition of viral particles of the *Adenovirus* type.

**14.** Use according to claim 10 of at least a α-lactalbumin derivative and/or its pepsin hydrolysate, and/or a β-lactoglobulin derivative and/or its pepsin hydrolysate, the esterifiable groups of which are methylated to at least 60%, for the manufacture of a composition allowing the infection capacity inhibition of viral particles of the *Poliovirus* type and/or other naked and enveloped viruses.

**15.** Use according to claim 10 of at least a pepsin hydrolysate of a α-lactalbumin derivative, and/or a β-lactoglobulin derivative, the esterifiable groups of which are methylated to at least 60%, for the manufacture of a composition allowing the infection capacity inhibition of viral particles of the *Cytomegalovirus* type.

**Patentansprüche**

**1.** Nichttherapeutische präventive Verwendung einer Zusammensetzung in einer Umgebung, die Viruspartikel enthält oder enthalten könnte, mit Ausnahme von in-vivo-Umgebungen zur wenigstens partiellen Hemmung der Infektionsmechanismen der Viruspartikel, wobei die Zusammensetzung wenigstens ein Protein, das aus α-Lactalbumin und/oder β-Lactoglobulin ausgewählt ist, und/oder ein Peptid, das von einem solchen Protein stammt, umfasst, bei denen wenigstens eine der veresterungsfähigen Gruppen durch Veresterung in Gegenwart von Methanol oder Ethanol modifiziert ist.

**2.** Verwendung gemäß Anspruch 1, wobei die Zusammensetzung wenigstens ein Derivat von α-Lactalbumin und/oder dessen Pepsin-Hydrolysat umfasst, bei dem die veresterungsfähigen Gruppen zu 50 bis 70% methyliert sind, zur Hemmung der Infektionsfähigkeit von Viruspartikeln des Acyclovir-empfindlichen Herpes-simplex-Typs.

3. Verwendung gemäß Anspruch 1, wobei die Zusammensetzung wenigstens ein Derivat von β-Lactoglobulin umfasst, bei dem die veresterungsfähigen Gruppen zu wenigstens 90% methyliert sind, zur Hemmung der Infektionsfähigkeit von Viruspartikeln des Acyclovir-resistenten Herpes-simplex-Typs.

4. Verwendung gemäß Anspruch 1, wobei die Zusammensetzung wenigstens ein Derivat von β-Lactoglobulin und/oder dessen Pepsin-Hydrolysat umfasst, bei dem die veresterungsfähigen Gruppen zu wenigstens 90% methyliert sind, zur Hemmung der Infektionsfähigkeit von Viruspartikeln des Adenovirus-Typs.

5. Verwendung gemäß Anspruch 1, wobei die Zusammensetzung wenigstens ein Derivat von α-Lactalbumin und/oder dessen Pepsin-Hydrolysat und/oder ein Derivat von β-Lactoglobulin und/oder dessen Pepsin-Hydrolysat umfasst, bei dem die veresterungsfähigen Gruppen zu wenigstens 60% methyliert sind, zur Hemmung der Infektionsfähigkeit von Viruspartikeln des Poliovirus-Typs und/oder anderer unbehüllter und behüllter Viren.

6. Verwendung gemäß Anspruch 1, wobei die Zusammensetzung wenigstens ein Pepsin-Hydrolysat eines Derivats von α-Lactalbumin und/oder eines Derivats von β-Lactoglobulin umfasst, bei dem die veresterungsfähigen Gruppen zu wenigstens 60% methyliert sind, zur Hemmung der Infektionsfähigkeit von Viruspartikeln des Cytomegalievirus-Typs.

7. Präventive Verwendung einer Zusammensetzung gemäß einem der Ansprüche 1 bis 6 als Desinfektionsmittel zur Behandlung von Oberflächen, zum Beispiel in Pulverform, enthalten in Antiseptika oder Detergentien.

8. Präventive Verwendung einer Zusammensetzung gemäß einem der Ansprüche 1 bis 6 als Mittel zur Bekämpfung von viralen Kontaminationen bei der Behandlung von Abwässern.

9. Präventive Verwendung einer Zusammensetzung gemäß einem der Ansprüche 1 bis 6 als antivirales Mittel, das auf der Oberfläche von Mundschutzgeweben oder von Schutzhandschuhen abgeschieden ist.

10. Verwendung wenigstens eines Proteins, das aus α-Lactalbumin und/oder β-Lactoglobulin ausgewählt ist, und/oder eines Peptids, das von einem solchen Protein stammt, bei denen wenigstens eine der veresterungsfähigen Gruppen durch Veresterung in Gegenwart von Methanol oder Ethanol modifiziert ist, zur Herstellung einer Zusammensetzung, die eine wenigstens partielle Hemmung der Infektionsmechanismen von Viruspartikeln gewährleistet.

11. Verwendung gemäß Anspruch 10 wenigstens eines Derivats von α-Lactalbumin und/oder seines Pepsin-Hydrolysats, bei dem die veresterungsfähigen Gruppen zu 50 bis 70% methyliert sind, zur Herstellung einer Zusammensetzung zur Hemmung der Infektionsfähigkeit von Viruspartikeln des Acyclovir-empfindlichen Herpes-simplex-Typs.

12. Verwendung gemäß Anspruch 10 wenigstens eines Derivats von β-Lactoglobulin, bei dem die veresterungsfähigen Gruppen zu wenigstens 90% methyliert sind, zur Herstellung einer Zusammensetzung zur Hemmung der Infektionsfähigkeit von Viruspartikeln des Acyclovir-resistenten Herpes-simplex-Typs.

13. Verwendung gemäß Anspruch 10 wenigstens eines Derivats von β-Lactoglobulin und/oder seines Pepsin-Hydrolysats, bei dem die veresterungsfähigen Gruppen zu wenigstens 90% methyliert sind, zur Herstellung einer Zusammensetzung, die die Hemmung der Infektionsfähigkeit von Viruspartikeln des Adenovirus-Typs erlaubt.

14. Verwendung gemäß Anspruch 10 wenigstens eines Derivats von α-Lactalbumin und/oder seines Pepsin-Hydrolysats und/oder eines Derivats von β-Lactoglobulin und/oder seines Pepsin-Hydrolysats, bei dem die veresterungsfähigen Gruppen zu wenigstens 60% methyliert sind, zur Herstellung einer Zusammensetzung, die die Hemmung der Infektionsfähigkeit von Viruspartikeln des Poliovirus-Typs und/oder anderer unbehüllter und behüllter Viren erlaubt.

15. Verwendung gemäß Anspruch 10 wenigstens eines Pepsin-Hydrolysats eines Derivats von α-Lactalbumin und/oder eines Derivats von β-Lactoglobulin, bei dem die veresterungsfähigen Gruppen zu wenigstens 60% methyliert sind, zur Herstellung einer Zusammensetzung, die die Hemmung der Infektionsfähigkeit von Viruspartikeln des Cytomegalievirus-Typs erlaubt.

| µg/ml | 1 | 2 | 3 | 4 |
|---|---|---|---|---|
| 4 | 71±6 | 64±6 | 47±5 | 67±6 |
| 20 | 82±7 | 65±6 | 72±7 | 82±6 |
| 100 | 85±5 | 89±5 | 94±6 | 85±5 |

<u>fig. 1</u>

fig. 2

fig. 3

fig. 4

fig. 5

fig. 6

fig. 7

fig. 8

fig. 9

fig. 10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5985275 A

- EP 0736102 A

**Littérature non-brevet citée dans la description**

- **Pal R. et al.** *Science,* 1997, vol. 278, 695
- **Struyf S. et al.** *J.Immunol,* 1998, vol. 161, 2672
- **Cota M. et al.** *Poli.G. PNAS,* 2000, vol. 97, 9162
- **Gordien E.** *J.Virol,* 2001, vol. 75, 2684
- **Arnold D. et al.** *Antiviral Res.,* Février 2002, vol. 52 (2), 158-8
- **SWART et al.** *Journal of Peptides Science,* Décembre 1999, vol. 5 (12), 563-576
- **OVERMANN et al.** *Antiviral Research,* 01 Juin 2003, vol. 59 (1), 23-33
- **FLORIS et al.** *Current Pharmaceutical Design,* vol. 9 (16), 1257-1275

- **M. Sitohy et al.** *International Journal of Biological Macromolecules,* 2001, vol. 29, 259-266
- **Fraenkel-Conrat H. ; Olcott H.S.** *J. Biol. Chem.,* 1945, vol. 161, 259-269
- **Mailliart ; Ribadeau Dumas.** *J.Food Sci.,* 1988, vol. 53, 743-745
- **Halpin, M.I. ; Richardson, T.** *J. Dairy Sci.,* 1985, vol. 68, 3189-3198
- **Bertrand-Harb, C. ; Chobert, J.M. ; Dufour, E. ; Haertlé, T.** *Sci. Aliments,* 1991, vol. 11, 641-652
- **Frister et al.** *Anal. Chem.,* 1988, vol. 330, 631-633
- **Mac Laren et al.** *Antivirale Res.,* 1983, vol. 3, 223-234